Europäisches Patentamt

European Patent Office

Office européen des brevets

(11) Publication number: **0 389 301**
**A2**

# EUROPEAN PATENT APPLICATION

(21) Application number: **90303159.9**

(22) Date of filing: **23.03.90**

(51) Int. Cl.⁵: **G01N 33/58, G01N 33/68,**
**//G01N33/74,G01N33/76**

(30) Priority: **24.03.89 US 328525**

(43) Date of publication of application:
**26.09.90 Bulletin 90/39**

(84) Designated Contracting States:
**AT BE CH DE DK ES FR GB GR IT LI LU NL SE**

(71) Applicant: **BIOGENEX LABORATORIES**
**4600 Norris Canyon Road**
**San Ramon California 94583(US)**

(72) Inventor: **Key, Marc E.**
**155 Cabro Court**
**Novato, California 94947(US)**
Inventor: **Gupta, Bipin C.**
**715 Catamaran, No. 2**
**Foster City, California 94407(US)**
Inventor: **Kalra, Krishan L.**
**4515 Kingswood Drive**
**Danville, California 94526(US)**

(74) Representative: **Harrison, David Christopher**
**et al**
**MEWBURN ELLIS 2 Cursitor Street**
**London EC4A 1BQ(GB)**

(54) **Reagent complex for immunoassay.**

(57) Methods and a reagent complex suitable for attaching detectable signals to specific-binding reagents, the complex comprising a first unlabelled antibody, wherein the first antibody has free specific-binding affinity for an analyte or an analyte-specific binding reagent; and a second antibody having specific-binding affinity for an Fc region of the first antibody, the second antibody being bound to the Fc region to form the complex and being labelled with a detectable signal.

EP 0 389 301 A2

# REAGENT COMPLEX FOR IMMUNOASSAY

The present invention is directed to reagents for use in immunoassays and other assays involving analyte-specific binding reagents, to techniques used to bind reporter groups to an analyte through a primary specific binding compound, and to the use of such reagents in assays and associated assay kits.

Immunoassays are well-known diagnostic techniques for the determination of the presence of antigenic analytes in body fluids, such as serum or urine, or in tissue samples. The clinical application of immunoassays has resulted in major advances in the diagnosis and classification of many physiological disorders. Immunoassay techniques are based upon the formation of a complex between the antigenic analyte being assayed and an antibody or antibodies in which at least one member of the complex may be labelled, for example, by a radioactive element, dye, or enzyme, which permits detection and/or quantitative measurement of the analyte.

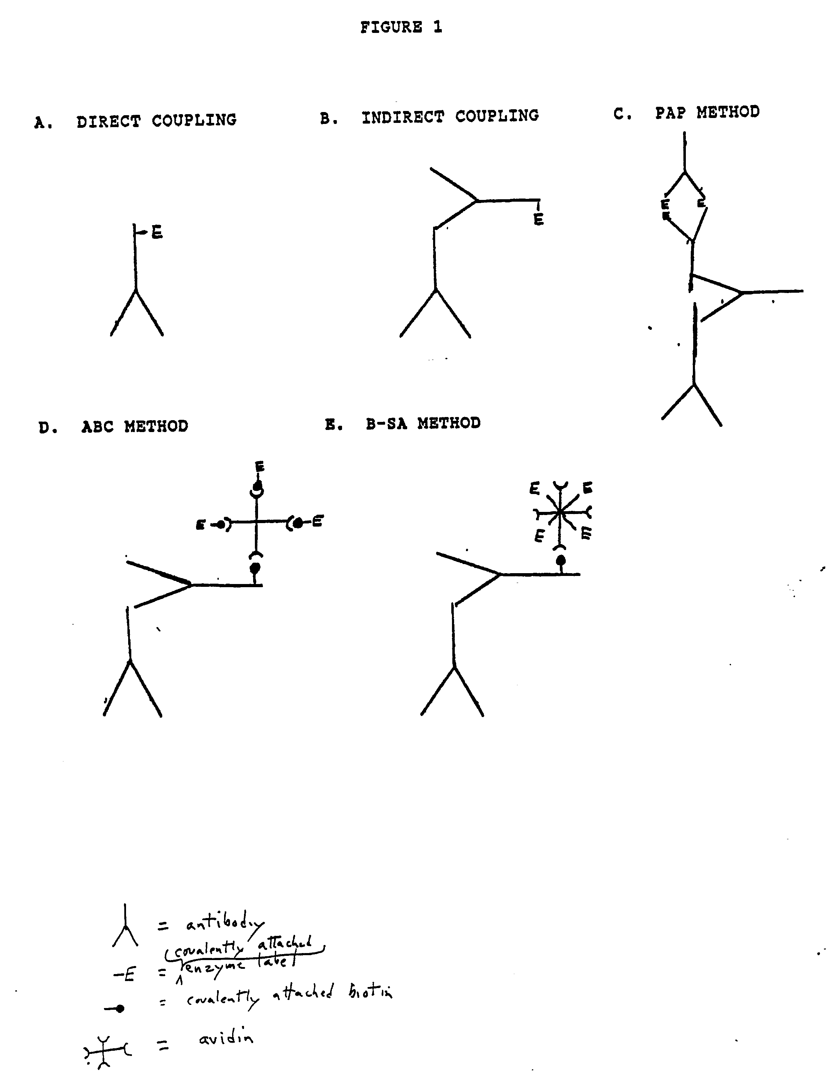

A popular method to visually localize cellular or tissue antigens is to couple a specific antibody (primary antibody), which reacts with the antigen of interest, with a label such as an enzyme. One of the first methods to involve coupling of an enzyme directly to the primary antibody was described in Nakane and Pierce, J. Histochem. Cytochem. (1966) 14:929-938. This was a "direct" assay using a single enzyme label on the primary antibody (Figure 1A). These "direct" methods proved to be inadequate for many applications because they lacked sensitivity. Furthermore, conjugating several enzyme molecules to the antibody impaired the specific-binding affinity of the antibody. To improve sensitivity, "indirect" labelling was introduced (Sternberger et al., J. Histochem. Cytochem. (1970) 18:315-333. In its simplest form the "indirect" method utilizes a second antibody, which is enzyme-labelled, that recognizes the primary antibody after the primary antibody has bound to the target analyte, thus leaving the primary antibody unaltered during the primary analyte-recognition event (Figure 1B). In later modifications of the "indirect" method, it was discovered that sensitivity could be further enhanced by an additional step used to join the primary antibody and the enzyme label. This step is commonly known as the "link" step because it links the primary antibody to the enzyme label. The "link" is a reagent, for example an antibody, which can bind to both the primary antibody of the first step and an enzyme, enzyme conjugate, or enzyme complex, thus linking the enzyme to the primary antibody.

Currently, there are three tissue staining systems which are commercially available and are popularly used for demonstration of antigens in tissues. All three methods employ a "link" step to take advantage of the added sensitivity that this step imparts. In these procedures the link is often called a secondary antibody to distinguish it from the first, or primary, antibody.

The first of these procedures is the peroxidase-antiperoxidase procedure (PAP; Sternberger et al. op. cit.) which was first introduced in 1970. According to this procedure, the tissue to be examined is first reacted with a primary antibody specific for the antigen of interest. A secondary antibody is then applied which can bind specifically with the primary antibody. The secondary antibody has two binding sites, one which binds to the primary antibody and one which is free to bind to the PAP complex. The PAP complex is composed of a mixture of peroxidase enzyme and antiperoxidase antibodies which form a peroxidase-antiperoxidase complex. The PAP complex is applied to the tissue after binding of the secondary antibody to the primary antibody where it is bound by the remaining binding site of the secondary antibody (Figure 1C). The sample is then treated with a reagent capable of reacting with the peroxidase to form a visually detectable colored compound.

A second commonly used immunological staining technique uses an avidin-biotin complex (ABC; Hsu et al. J. Histochem. Cytochem. (1981) 29:577-580). The high affinity of avidin for biotin ($Ka = 10^{15}$ $M^{-1}$) is employed to form a complex which comprises avidin and a biotinylated enzyme, usually peroxidase. The avidin-biotin complex has enhanced sensitivity because avidin possesses four binding sites for biotin. Therefore this complex has been found to contain more enzyme than other methods (Hsu et al. Am. J. Clin. Pathol. (1981) 75:734-738). According to the ABC method, a tissue is first reacted with a primary antibody specific for the cell-surface marker of interest. Next, a secondary antibody, specific for the primary antibody, is applied to the tissue. The secondary antibody is also bound to several biotin molecules. The tissue is then treated with an avidin-biotin enzyme complex which becomes bound to the biotin of the secondary antibody (Figure 1D). The tissue is then treated with a reagent capable of reacting with the enzyme to produce a colored reaction product.

The third commonly used immunological tissue staining method is the biotin-streptavidin (B-SA) method. First, a primary antibody is added to the tissue sections; then a biotinylated secondary antibody is added. Following these two steps, a solution of streptavidin-enzyme conjugate is added to the tissue section. The enzyme conjugate binds to the biotinylated secondary antibody through a biotin/streptavidin interaction (Figure 1E). This is followed by the addition of a reagent that reacts with the enzyme to form a colored reaction product.

Although individual researchers have reported differences in sensitivity among these three techniques, there is no general agreement that one system is definitely superior to the others. All three systems have in common the capability of binding multiple enzyme labels to each primary antibody. A feature which is common to all three methods is the utilization of a secondary (link) antibody which can bind at least one enzyme complex, each enzyme complex containing one or more enzyme labels. These secondary antibodies are added to the material being stained after the primary antibody has reacted with the cell surface antigen that is its specific binding target.

The above discussion, along with Figure 1, illustrates five ways in which an enzyme label can be linked to a specific analyte. Although these examples have been discussed in terms of staining tissue antigens, they are equally applicable to other enzyme immunoassays such as the ELISA (enzyme-linked immunosorbant assay).

In a typical ELISA, individual wells of a multi-well plate are coated with an antibody specific for the analyte to be measured. Next, an unknown sample suspected of containing the analyte is added to the well, and, if present, the analyte is immobilized by the antibody to the well. Subsequent detection of the immobilized antigen can employ any of the five detection methods discussed above and illustrated in Figure 1. For example, the ABC and B-SA methods of analyte detection have been described in U.S. Patent 4,535,057 for the detection of Herpes simplex virus.

Despite the success of these procedures, there presently exists a need for even more sensitive immuno-assays so that lower concentrations of antigen can be detected.

The present invention provides a reagent complex suitable for use in analytical assys that employ a detectable signal attached to a specific-binding compound, comprising a first unlabelled antibody, wherein said first antibody has free specific-binding affinity for (a) an analyte or (b) an analyte-specific binding reagent, and a second antibody having specific-binding affinity for an Fc region of said first antibody, said second antibody being bound to said Fc region to form said complex and being labelled with a detectable signal. The reagent complex can be used to attach a detectable signal to an analyte or to a specific-binding reagent that is used to detect the presence of analyte in an analytical assay, particularly an immunoassay or an immunostaining technique.

The invention will be better understood by reference to the following detailed description of the invention when considered in combination with the accompanying drawings that form part of the present specification, wherein:

Figure 1 shows a series of schematic diagrams of known products of step-wise, specific-binding reactions.

Figure 2 shows a series of schematic diagrams of preformed antibody complexes that can be used in the practice of the present invention.

Figure 3 is a schematic diagram showing use of two complexes of the invention in an exemplary ELISA sandwich assay.

Figure 4 is a graph showing limits of antigen detection for an assay using a complex of the invention and a second assay using a comparative technique to connect the detectable label to the antigen.

Figure 5 is a graph showing limits of antigen detection for a second assay using a complex of the invention that includes a primary antibody as part of the complex and a second assay using a comparative technique to connect the detectable label to the antigen.

It has been discovered that sensitivity of immunoassays in general, and immunohistology in particular, can be significantly enhanced by using a pre-formed complex of two antibodies in the assay, either as a link reagent to attach a detectable label to a primary antibody or other analyte-specific binding reagent or as a complex containing a primary antibody that reacts directly with the analyte. As will be shown by the following disclosure, such a pre-formed reagent complex can increase sensitivity by approximately 5 to 50 fold over existing methods.

The soluble, pre-formed complex comprises a first, unlabelled antibody and a second antibody that specifically binds to the Fc portion of the first antibody. There are two possibilities for the specific-binding affinity of the first antibody: (1) it can be an analyte-specific binding reagent itself, i.e., a primary antibody that reacts with the analyte, in which case the complex is referred to as a "primary complex," or (2) it can serve to link a different analyte-specific binding reagent to a detectable label through the second antibody, in which case the complex is referred to as a "link complex." In both case, however, the complex is formed between the first and second antibody before the first antibody binds to its target. A reference in this specification to a complex without modification by "primary" or "link" can refer to either type of complex, unless such limitation is apparent from the context of the reference.

An important aspect of the present invention is that the second antibody binds only to the Fc portion of the first antibody. If such antibodies are selected for use as the second antibodies of the complex, the specific-binding properties of the first antibody are retained without significant, usually without measurable inactivation. The complex can thus be formulated in a solution such that it retains its solubility and biological activity for substantial periods of time. This long shelf life of the link complex allows it to be used in assay kits. The complex can be applied to a wide variety of immunological assays, for example, immunohistology, ELISA, and RIA.

When the first member of the complex antibody pair (first antibody) is an antibody directed against an analyte or analyte-specific binding reagent (a primary antibody), such as an immunoglobulin of another species, the entire complex takes on the binding characteristics of the first antibody in that the entire will bind to the immunoglobulin of that particular species. In other words, the specific binding characteristics of the complex are determined by the first member of the complex antibody pair. Here "antibody pair" does not mean that only two antibodies can be present, as discussed in more detail below. Rather there is a binding relationship between the two antibodies so that the second antibody binds to the Fc region of the first antibody.

The second member of the complex antibody pair comprises an antibody to which is bound (covalently or non-covalently) some form of detectable signal such as an enzyme, fluorochrome, isotope, biotin, or avidin, which can be detected either directly or indirectly by a subsequent reaction. The complex so formed between the two antibodies can contain substantially more copies of the signal than could be directly attached to the first antibody alone. Therefore, the enhanced sensitivity of the complex is achieved by formation of a soluble complex which contains multiple copies of a particular signal.

In one embodiment of the present invention, the specific signal is biotin, which, in itself, is not the ultimate signal but acts as a link to bind a streptavidin-enzyme conjugate that produces the actual signal on reaction with an enzyme substrate.

A particularly preferred embodiment of present invention provides an assay method which utilizes a soluble, pre-formed link complex comprising a pair of antibodies such that the first member of the pair is an unlabelled antibody specific for a specific binding compound that provides the recognition of the analyte, and the second member is a biotinylated antibody molecule specific for the Fc portion of the unlabelled antibody. Furthermore, it has been discovered that a particularly useful complex can be made when the molar ratio of the first member to the second member is in the range 1:2 to 1:5, particularly 1:2.5 to 1:3.6, especially about 1:3.

The present invention is especially useful when the specific binding molecule that provides recognition of the target analyte ( referred to herein as the "analytical specific binding reagent") is itself an antibody. The first member of the complex is then an antibody against the immunoglobulin of another species, for example, goat anti-mouse IgG, heavy and light chain specific. This member imparts to the complex the desirable characteristic of selective binding to mouse immunoglobulin. The second member of the pair is, for example, a biotinylated antibody specific for the Fc portion of the first member, for example, a rabbit anti-goat IgG Fc specific. The second member introduces multiple copies of the biotin molecule into the link complex without interfering with the binding properties of the first member.

As used herein, "label," "signal," "detectable label," and "detectable signal" all refer to molecules, isotopes, and the like whose presence can be detected in a sample so that if the label is physically linked to an analyte, the presence of the analyte in the sample can be determined. The method of the invention is applicable to assays utilizing any detectable label, such as enzymes and coenzymes, fluorophores, spin labels, chemiluminescent and bioluminescent labels, and radioisotopes. Preferably, however, an enzyme label is used, and the invention will hereafter be described with reference to enzyme labels. It is understood, however, that other labels, such as those described above, can be employed. In all cases the signal can be incipient, i.e., a molecule can be present which, while not itself detectable, is capable of binding specifically with another molecule that provides the detectable signal. For example, the primary label can be biotin, and a streptavidin molecule covalently bound to an enzyme conjugate can be used as the secondary label which reacts with the first label to provide the complex that is actually detected.

"Link antibody complex" or "link complex" refers to the entire combination of molecules that links the labels to the analyte-specific binding reagent by physically binding to both the analyte- specific binding reagent and the label. The link complex may contain, in addition to the antibody pair previously discussed, specific-binding molecules, preferably biotin, which can specifically bind partners of the specific-binding molecules that are directly bound or attached to the labels, such as avidin- or streptavidin-conjugated labels. Similar additional molecules can be present when the complex is a primary complex rather than a link complex.

"Analytical specific binding reagent" as used herein refers to an antibody or other specific-binding molecule with specific reactivity for a particular analyte which is to be detected or measured. When this molecule is an antibody, it is often referred to as the primary antibody. A primary antibody may be either monoclonal or polyclonal and may be derived from any species of animal. However, the present invention may also be applied to detection of a biospecific macromolecule using a nucleic acid probe, lectin, hormone receptor, or other molecule exibiting specific binding character. For example, nucleic acid probes can be utilized in such well-known assays as "Northern" and "Southern" assays. See, e.g., Nucleic Acid Hybridization: A Practical Approach (Hames and Higgins eds. 1985).

"Specific-binding affinity" refers to the ability of a molecule to selectively react with an analyte in comparison to other molecules that are present in a sample that contains (or potentially contains) the analyte. Analyte-specific binding reagents have well-know and defined regions on their surfaces at which the specific binding takes place (e.g., hydrogen bonding between bases in nucleic acid probes and between antigens and variable regions of antibodies). Affinities of at least $10^{-7}$ $M^{-1}$ are desirable, preferably $10^{-9}$, more preferably $10^{-10}$. Lower affinities are permitted when there is little or no interference caused by binding between the analyte-specific binding reagent and non-analyte components of the assay medium.

"Avidin or analog thereof" refers to the originally discovered avidin molecule or to any related peptide (e.g., streptavidin) which has subsequently been discovered. These avidin-type peptides are characterized in that they have similar, although not identical, amino acid structures and share the ability to complex biotin at exceptionally high affinity (at least $10^{-12}$, preferable $10^{-15}$, $M^{-1}$). The present invention will be discussed in terms of avidin or streptavidin, but it is to be understood that the invention can be practiced with any suitable avidin analog. Streptavidin is preferred for immunostaining applications since it exhibits very little non-specific binding to normal tissues, such as kidney, liver and brain tissue, and mast cells.

In one preferred embodiment, the present invention employs a link complex comprising 1) an antibody which binds specifically to the immunoglobulin of another species, and 2) an antibody which binds to an epitope on the Fc portion of the first antibody molecule. Additionally, the second antibody will also be bound or attached to additional specific-binding molecules, such as biotin, in order that a label can be later bound to the complex, or it will be directly attached to a detectable label.

In another preferred embodiment, a primary complex of the invention contains, in addition to the first and second antibody, a third antibody also bound through its Fc region to the second antibody. Typically the first and third antibodies will be derived from a first host species (e.g., mouse) and the second antibody from a second host species (e.g., goat anti-mouse Fc specific IgG). By derived is meant any process that produces an antibody having immunological characteristics associated with a particular species, whether the antibody is obtained by in vivo immunization and antiserum collection, fusion of cell lines to produce a hybridoma, techniques of genetic engineering used to produce a chimeric antibody (e.g., having variable and constant regions derived from different species), or any other process. Depending on the specific-binding affinity of the third antibody, different types of complex are obtained (all still classified as primary complexes). If the third antibody has specific-binding affinity for a second eptiope (such as a second cell-surface antigen) the complex is referred to as being bi-specific. If the third antibody has specific-binding affinity for the label or a complex containing or capable of later binding the label, the complex is referred to as a bi-functional complex, to indicate its ability to function both as a primary reagent and a means of easily binding the label. The third antibody functions in the same manner in the bi-functional embodiment as biotin in link complexes discussed above; i.e., as an incipient label.

The antibodies employed in the link of the present invention can be either monoclonal or polyclonal antibodies. "Monoclonal antibodies" refers to a composition of antibodies produced by a clonal population (clone or cell line) derived through mitosis from a single antibody-producing cell. A composition of monoclonal antibodies is substantially free of other antibodies, in that it is free of antibodies that are not produced by cells originating from the clonal population. "Substantially free" of other antibodies preferably means approximately 5% (w/w) or fewer, more preferably 2% or fewer, contaminating antibodies are present in the composition. The general procedures for producing compositions comprised of monoclonal antibodies, including production of the cell lines, which produce such compositions, are well known in the art. See, e.g., Gerhard et al., Proc. Natl. Acad. Sci. USA (1978) 75:1510; Cozbar et al., Proc. Natl. Acad. Sci. USA (1982) 79:6651; Jonak et al., Hybridoma (1983) 2:124; Kozbor et al., Immunology Today (1983) 4:72; Shulman et al., Nature (1982) 276:269; Foung et al., Proc. Natl. Acad. Sci. USA (1983) 79:7484. Any appropriate technique of immortalizing a primary B-cell producing the antibody of interest can be employed, including, but not limited to, fusion with a myeloma cell, transformation with oncogenic DNA, and transfection with Epstein-Barr virus. It is preferred to employ mammalian monoclonal antibodies such as murine monoclonals. As used herein, "polyclonal" antibody refers to conventional polyclonal antibodies (e.g., prepared from antiserum), or a mixture of monoclonal antibodies. Polyclonal antibodies can be readily isolated from the pooled sera of antibody-positive individuals by standard techniques or from antisera produced in a mammal of choice, typically, a mouse, rabbit or goat. In preparing second antibodies that are specific for the Fc region of the first antibody, the immunogen is typically a collection of Fc region fragments obtained by enzymatic cleavage of an antiserum or monoclonal antibody collection obtained from the same species as the immunological host used to prepare the first antibody. Fc-specific antibodies are also commercially available and can readily be employed to form a complex of the invention.

When the complex of the invention is a link complex, it is employed after a primary antibody recognizes an epitope on the analyte or other analyte-specific binding reagent has been incubated with the sample. Further discussion refers to primary antibodies for simplicity. The pre-formed link complex is then incubated with the primary-antibody-contacted sample and binds to any primary antibody found therein. For example, a test sample is first incubated with an insoluble anti-analyte antibody, thereby immobilizing any analyte present in the sample. Second, the immobilized analyte is incubated with a soluble anti-analyte antibody (primary antibody) so that the soluble antibody becomes immobilized by binding to any insoluble analyte. Third, the link complex is incubated with the immobilized sample so that the link complex binds to any of the soluble antibody that has become immobilized. Since many such soluble or primary antibodies used in assays are murine monoclonal antibodies of class IgG, a common antibody for use in a link complex according to the present invention would be an anti-murine IgG antibody.

The selection of the appropriate antibodies and/or other specific-binding molecules will depend upon the type of assay intended and is within the skill of the art.

The antigen or analyte which interacts with the primary antibody may be monoepitopic or polyepitopic material. It may be selected without limitation from materials such as drugs, metabolites, natural products, pesticides, chemicals, and contaminants of air and water. Examples include drugs such as digoxin, digitoxin, phenytoin, theophylline, gentamicin, and tobramycin; alkaloids such as morphine, heroin, cocaine, ergot alkaloids, and the like; steroids such as the steroid hormones including estrogens and androgens, for example estriol, and anti-inflammatory steroids, for example cortisol; lactams such as the barbiturates including phenobarbital; aminoalkylbenzenes such as the amphetamines; vitamins; protaglandins such as $F_2$alpha and E; antibiotics and the like; short peptide sequences or amino acids such as thyroxine, triiodothyronine and oxytocin. Representative pollutants and pesticides include PCB, dioxin, halogenated biphenyls, carbamates, thiophosphites, phosphate esters and their metabolites. Such materials can range in molecular weight from about 50 to about 2000.

The antigen or target molecule can also be a polymeric material such as a protein or other polyamino acid, a polynucleic acid or a polysaccharide. Typical protein analytes can be taken from any of the classes of proteins including, without limitation, globulins, albumins, lipoproteins, glycoproteins, histones and the like, hypersensitive proteins including albumin, the immunoglobulins such as IgE, fibrinogen, transferrin, the various complement factors, tumor markers like CEA (carcinoembrionic antigen) and PAP, various blood clotting factors, and protein hormones including $\beta$-hcG, FSH, gastrin, HG and prolactin, insulin, thyrotopin, gonadotropin and the like. Example of polysaccharides include those derived from microorganisms such as those associated with various species of Salmonella, Streptococcus, and Klebsiella. Other targets include, without limitation, pathogens such as hepatitis A and B, rubella, and human immunodeficiency virus.

The foregoing list is intended to be a brief outline. It is to be recognized that other analytes, such as are listed in more detail in the art (see, for example, U.S. Pat. No. 4,193,983, columns 7-11 incorporated herein by reference), can be used in conjunction with analyte-specific binding reagent and the reagent complex provided by this invention.

When the complex of the invention is a link complex, formation of the complex begins with selection of the appropriate primary antibody or antibodies. The link complex is then formulated so that the first unlabelled antibody will react specifically with the primary antibody. For example, if the primary antibody is of mouse origin, then goat anti-mouse IgG can be used as the first antibody of the link complex.

The second component of the complex (both the link complex and the primary complex) comprises an antibody specific for the Fc region of the first antibody, for example, rabbit anti-goat IgG Fc specific.

Preferably, the second antibody is also biotinylated according to standard procedures. Commercial reagents for biotinylating proteins are available from chemical/biochemical supply houses. A labelling reagent capable of specific-binding to biotin is then utilized, thereby introducing a detectable signal to the complex. A preferred embodiment employs enzymes covalently bound to streptavidin. Preferred enzyme labels include, but are not limited to, glucose oxidase, horseradish peroxidase, $\beta$-galactosidase, and alkaline phosphatase.

The label allows a signal to be measured which is related to the amount of analyte in a sample. Common signals are radioactive emissions (when radio-isotopes are used), optical density (e.g., enzyme reactions), and fluorescence (fluorescing compounds). It is preferred, as mentioned above, to employ a signal such as optical density (or color intensity) which does not require the handling of radioactive materials. Numerous enzymes/substrate combinations are known in the immunoassay art that can produce a suitable signal, such as color intensity or development. See, e.g., U.S. Pat. Nos. 4,323,647 and 4,189,496, the disclosures of which are incorporated herein by reference.

The complex of the present invention is prepared in a buffer wherein the pH is maintained at an immunologically permissive pH. The biological activity of an antibody can be generally maintained at a pH range of 5.0-9.0, preferably 7.0-8.0. Phosphate buffered saline (PBS) at pH 7.6 is therefore a preferred buffer, and the buffer may include stabilizing agents such as, for example, polyethylene glycol (PEG), polyvinyl chloride, and inert proteins such as gelatin, casein, or albumin. These agents are helpful in the reagent solution to prevent aggregation of the complex.

Since the concentration and activity of all complex components vary among different lots of the same product, it is useful to determine optimal ratios and concentrations for each of these component lots before manufacturing the complex. The optimal ratio of the first binding pair to the second binding pair can be determined in the manner shown in Example 1 below. Then, using this ratio, the optimal concentration is determined by preparing small batches of the complex. The optimal concentration can be determined empirically be titration as described below and in the accompanying examples (see Example II). However, there is no requirement that the invention be practiced solely under optimal conditions.

The reagent solution containing the link complex of the present invention can be readily incorporated into standard ELISA procedures, as well as into Western blotting procedures. These two procedures are quite well known in the art and are briefly summarized below. Illustrative protocols for the use of the link complex of the invention are provided in the accompanying examples.

The ELISA protocol described in this paragraph is a typical prior art protocol useful for assaying a hybridoma population for clones producing the antibody of interest. Generally, an ELISA procedure using the sequential method of forming the biotinylated antibody and streptavidin-enzyme conjugate begins by coating 96-well, polystyrene, enzyme-immunoassay plates with a specific amount of antigen to detect the presence or absence of murine monoclonal antibody in tissue culture supernatants. Supernatant media from hybridomas producing varying levels of antigen-specific monoclonal antibody are added to each well and incubated. Monoclonal antibody that is present in the media binds to the antigen coated on the plates. Then, after a brief washing, biotinylated secondary antibody specific to all subclasses of murine monoclonal antibody is added and allowed to incubate in the wells. This step detects varying levels of murine monoclonal antibody that are bound to antigen in the previous step. Subsequent to a brief washing which removes unbound antibody, a streptavidin-peroxidase conjugate is added and incubated in the wells. The streptavidin-peroxidase conjugate will bind to any biotinylated antibody introduced in the previous step. After a brief washing, a soluble chromogenic substrate is added. Any bound peroxidase which remained converts the substrate to a specific intensity of color which provides a quantitative measure of murine monoclonal antibody present in the original culture supernatants.

The methodology of an ELISA (or other immunoassay) procedure according to the present invention using a link complex is generally the same, except that the pre-formed link complex is added instead of adding a murine-specific biotinylated secondary antibody.

In a typical prior art Western blot, varying concentrations of purified antigen are electrophoresed and transferred under constant voltage to a nitrocellulose membrane. The levels of antigen are detected by adding a biotinylated murine monoclonal antibody and allowing it to incubate on the membrane. Varying levels of murine monoclonal antibody is bound to the antigen in the previous step. After a brief washing to remove unbound antibody, a streptavidin-peroxidase conjugate is applied and incubated on the membrane. The streptavidin-peroxidase conjugate will bind to any remaining biotinylated antibody introduced in the previous step. After a brief washing, chromogenic substrate is added. Any bound peroxidase from the previous step converts the substrate to an insoluble precipitate which can be detected by the unaided eye.

The method of the present invention as applied to Western Blot Analysis is generally the same as the above-mentioned with the exception that instead of adding a murine-specific biotinylated secondary antibody, the pre-formed link complex is added.

The present invention provides assay test kits which advantageously employ the improved reagent solution described above. In general, these kits will contain a premeasured amount of the link complex along with other components, that depend upon the assay of interest. For example, optional components can include an immobilized analyte or immobilized anti-analyte antibody, for use in competition or sandwich assays, respectively. If the detectable label is an enzyme, the kits will also generally include a premeasured amount of the substrate which reacts with the enzyme. It is also conventional to include a wash solution, usually a buffered saline solution (e.g., PBS), in the kit.

Antibodies or antigen can be immobilized on an appropriate solid test support by an appropriate technique. The solid test support can be any suitable insoluble carrier material for the binding of antibodies or antigens in immunoassays. Many such materials are known in the art, including but not limited to nitrocellulose sheets or filters; agarose, resin, plastic (e.g., PVC, polystyrene) or metal beads; plastic vessels; and the like. Many methods of immobilizing antibodies and antigens are known in the art. See, e.g., Silman et al., Ann. Rev. Biochem. (1966) 35:873; Melrose Rev. Pure and Appli. Chem. (1971) 21:83; Cuatrecasas et al., Meth. Enzym. (1971) Vol. 22. Such methods include covalent coupling, direct adsorption, physical entrapment, and attachment to a protein-coated surface. In the latter method, the surface is first coated with a water-insoluble protein such as sein, collagen, fibrinogen, keratin, glutelin, etc. The antibody is attached by simply contacting the protein-coated surface with an aqueous solution of the antibody and allowing it to dry.

In general, the test support has a receptacle adapted to receive a fluid test sample. Usually the receptacle has a volume in the neighborhood of about 1 ml to about 0.25 ml, or less. The surface of the receptacle which comes into contact with the fluid test samples has the immobilized antibody or antigen thereon. In a preferred embodiment, the surface of the receptacle which contacts the fluid test sample is also coated with a protein which is non-reactive with the other components of the test sample and reagents. This minimizes non-specific binding of the analyte of interest to the test support.

Any combination of support and binding technique which leaves the antibody or antigen immunoreactive, yet sufficiently immobilized so that it is retained with any bound antigen during a washing, can be employed in the present invention. A preferred solid test support is a conventional plastic immunoassay plate or microtiter plate with wells of about 250 $\mu$l volume coated with antibody or antigen. The wells are then coated with blocking protein (e.g., gelatin or bovine serum albumin).

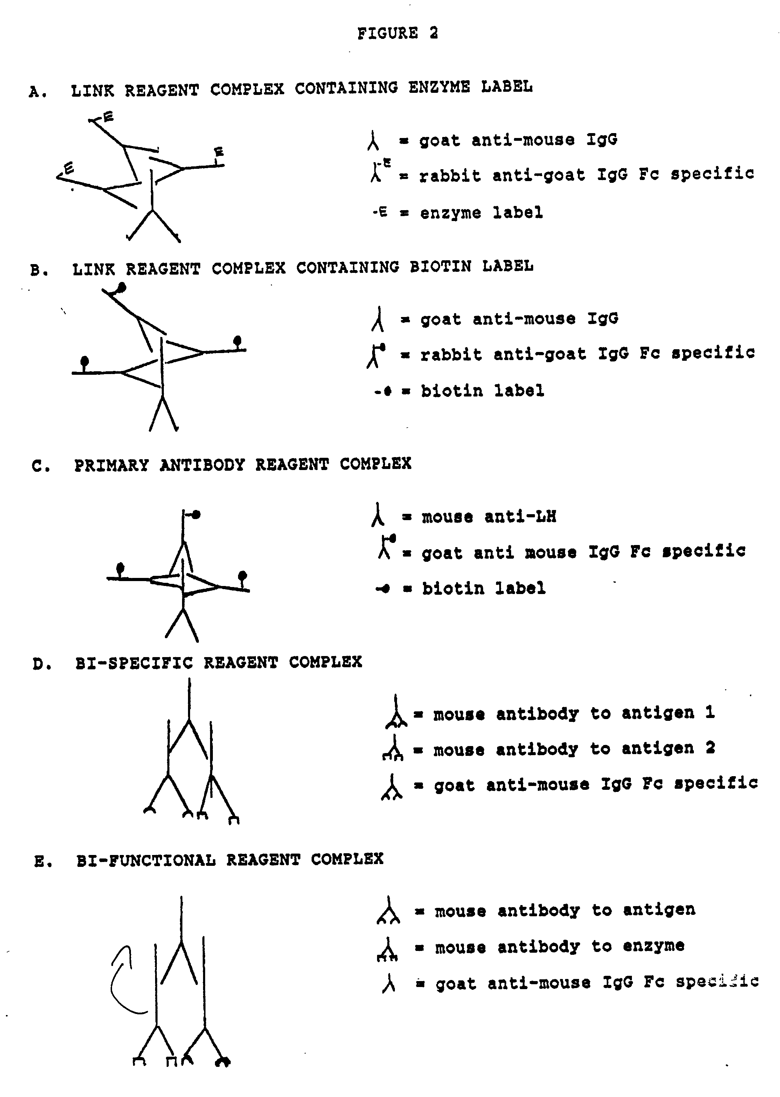

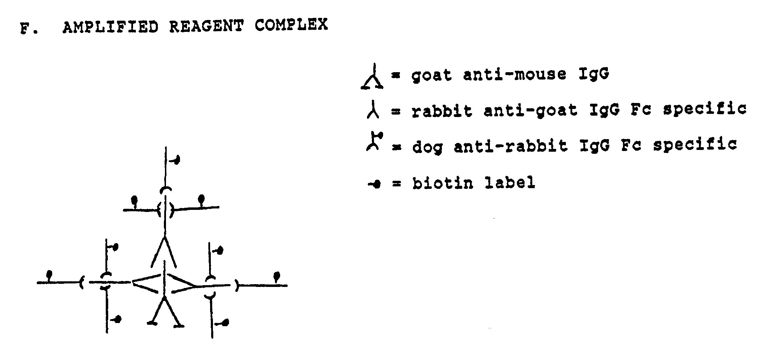

A number of different embodiments of the invention are show in schematic diagrams in Figures 2A-2F. Figure 2A shows a schematic diagram of one embodiment of the invention in which a link complex is used to attach an enzyme label to a primary antibody. Figure 2B depicts a link complex of the invention used to attach biotin molecules to a primary antibody.

Most of the specific examples above are directed to link complexes as opposed to primary complexes. This should not imply that primary complexes are of less interest. As previously discussed in general, the primary complex is prepared with a primary antibody specific for a particular analyte of interest. In all other respects this primary complex is similar to the previously described link complex. According to this embodiment, the primary complex would be reacted directly with the analyte, thereby binding a detectable signal, such as biotin, to the analyte without the necessity of an additional link step. For example, a primary complex could be prepared where the first binding partner is a murine monoclonal antibody to an analyte such as luteinizing hormone, and the second binding partner is a biotinylated goat anti-mouse IgG Fc-specific. This primary complex would then bind directly to LH, thereby binding the biotin label to the LH analyte. Such a complex is shown in Figure 2C.

In other embodiments of the present invention, various other types of complexes can be formed. Each complex would have in common that the second binding partner would be an antibody directed against the Fc region(s) of the other binding partner(s). One such reagent complex is a bi-specific primary complex (see Figure 2D) where two antibodies of different specificities (i.e., recognize different antigens or epitopes) are joined together by a common anti-Fc binding antibody. If the first two antibodies are murine monoclonal antibodies, then the joining antibody could be goat anti-mouse IgG Fc-specific.

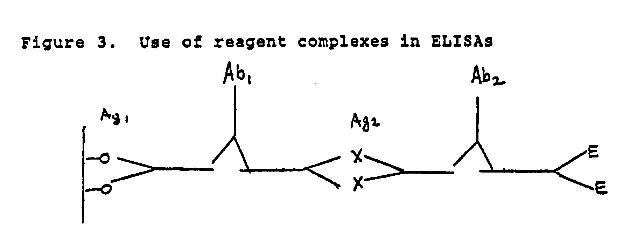

Such a bi-specific antibody could be used in an ELISA as illustrated in Figure 3. The bi-specific antibody ($Ab_1$) could bind simultaneously to $Ag_1$ which is bound to the surface of a well, and to $Ag_2$ which is the analyte to be measured (Figure 3).

In bi-functional primary complexes, two antibodies are joined by a common anti-Fc binding antibody. One of the two antibodies with free binding sites is directed against the analyte to be measured, and the second is directed against a detectable label such as an enzyme. The bi-specific primary complex could react with analyte (Ag) and enzyme (E) simultaneously, thereby directly linking enzyme to analyte. This concept is illustrated in Figure 2E. Such a bi-functional primary complex could be used in an ELISA as illustrated in Figure 3. $Ab_2$ is the bifunctional primary complex and can bind simultaneously to both the analyte being measured ($Ag_2$) as well as the enzyme label (E).

In an embodiment referred to as the amplified link concept, a reagent complex similar to that shown in Figure 2B is subsequently reacted with a second anti-Fc antibody which can bind specifically to the Fc-region of the first anti-Fc antibody. If the second anti-Fc antibody contains a detectable signal, such as biotin, the incorporation of additional detectable signals into the reagent complex would increase its sensitivity for detecting analyte.

Many other embodiments of the reagent complex principle can be envisioned, all having in common the aggregation of an antibody or group of antibodies (each having similar Fc regions) by another antibody which can specifically bind to the Fc regions of these antibodies causing the formation of a stable complex.

The above illustrations are only some of the possibilities, and are not meant to be all inclusive.
The following examples are offered by way of illustration and not by way of limitation.

## EXAMPLES

### Reagents

Goat anti-mouse IgG (H and L) were purchased from Jackson Immuno Research Laboratories (West Grove, PA; Product No. 115-6544). Biotinylated rabbit anti-goat IgG Fc specific was obtained from BiosPacific, Inc. Streptavidin-conjugated calf intestine alkaline phosphatase was purchased from Jackson Immuno Research. These first three reagents were purchased as lyophilized preparations. They were reconstituted according to manufacturer's specifications and stored at $4^\circ$ C until use.

ACS-grade potassium phosphate (anhydrous, di-and monobasic) and ACS-grade sodium chloride (Product Numbers 0705, 0337, and 0241, respectively) were purchased from Amresco (American Research Products Co., Solon, Ohio). Phosphate-buffered saline (PBS) was prepared from these reagents as a 25-fold concentrate consisting of 0.367 M $KH_2PO_4$, 0.215 M $K_2HPO_4$, 3.375 M NaCl in deionized $H_2O$. The pH was adjusted, if necessary to 6.60 ± 0.05 with 3 N NaOH or 3 M $H_3PO_4$. This concentrate was diluted to working strength with deionized $H_2O$ and the pH was adjusted, where necessary, to 7.60 ± 0.02 with 3 N NaOH before use. Working-strength PBS consisted of final concentrations of 14.7 mM $KH_2PO_4$; 8.6 mM $K_2HPO_4$; and 133 mM NaCl.

## EXAMPLE I

### Determination of Optimal Ratios for Link Reagent Complex

The sensitivity and stability of the link complex is dependent upon the particular ratio of the first and second binding pairs that comprise the reagent. To determine the optimal ratio of reactants, 20 ug of goat anti-mouse IgG (first binding pair) was added to each of 6 tubes which contained 1.0 ml of PBS and 1% bovine serum albumin. Next, biotinylated rabbit anti-goat IgG Fc-specific (second binding pair) was added to the tubes such that the molar ratio of the first binding pair to the second binding pair was 1:1, 1:2, 1:3, 1:4, 1:5, or 1:6 in tubes 1 through 6 respectively. This series of reagent complexes was then tested for staining on conventionally prepared paraffin-embedded tissues. After deparaffinization and hydration, the 5 micron tissue sections were incubated with an appropriate primary antibody under conditions established for that antibody and tissue. After a brief rinse with PBS, each reagent complex was applied to a tissue section and incubated at room temperature for 20 minutes. After a 5 minute wash in PBS, a solution containing 1 μg/ml of streptavidin-alkaline phosphatase in PBS with 1% bovine serum albumin was added to each tissue section and incubated for 20 minutes. After a 5 minute wash in PBS, color was developed for alkaline phosphatase by incubating for 30 minutes with a solution of 2 mg/ml Fast Red TR and 0.2 mg/ml naphthol AS MX phosphate (both from Sigma Chemicals) dissolved in 0.1 M Tris-HCl, pH 8.2. After counterstaining and coverslipping, the slides were graded on a scale of 0 to 4.5 as follows. For purposes of the present invention, a 0.0 to 4.5 grading scale is used throughout the following examples.

| Score | Definition |
|---|---|
| 4.5 | Maximum possible stain, to the point of obscuring cellular detail. |
| 4.0 | Extremely good stain; strong but permitting visualization of cellular detail. |
| 3.5 | Very good staining, with good contact between specific immunostained features and background. |
| 3.0 | Staining good but lighter than above, with contrast adequate. |
| 2.5 | Staining somewhat washed out; contrast weaker. |
| 2.0 | Stain weak, still somewhat distinguishable. |
| 0.5-1.5 | Stain barely discernible from background. |
| 0 | No discernible stain. |

The ratio yielding the best staining was then chosen as having the optimal ratio of the two binding pairs. The results from this test are summarized below.

TABLE 1

| Staining Intensity of Link Complex at Various Ratios of the Two Binding Pairs | | | |
|---|---|---|---|
| Link Complex | | | |
| Binding Pair 1 | Binding Pair 2 | Ratio | Staining of Insulin In Normal Pancreas |
| 20 $\mu$g | 10 $\mu$g | 1:.5 | 2.0 |
| 20 $\mu$g | 20 $\mu$g | 1:1 | 3.0 |
| 20 $\mu$g | 40 $\mu$g | 1:2 | 4.0 |
| 20 $\mu$g | 60 $\mu$g | 1:3 | 4.5 |
| 20 $\mu$g | 80 $\mu$g | 1:4 | 4.0 |
| 20 $\mu$g | 100 $\mu$g | 1:5 | 4.0 |
| 20 $\mu$g | 120 $\mu$g | 1:6 | 3.5 |
| Binding Pair 1 = goat anti-mouse IgG Binding Pair 2 = biotinylated rabbit anti-goat IgG Fc specific | | | |

These particular results indicate that a particular ratio of first binding pair to second binding pair of about 1:3 is optimal. Other ratios may not be optimal because when the second binding pair is reduced, less biotin is incorporated into the complex, and when the second binding pair is increased, the complex becomes unstable because of its large size. However, other ratios can be used when less stringent staining requirements are present.

EXAMPLE II

Determination of Concentration of Link Complex

To determine the concentration of link complex, a dilution series of link complex is made in a solution of 0.01 M potassium phosphate buffer, pH 7.6; 0.132 M NaCl; 1.0% (w/v) bovine serum albumin; and supplemented with either 0.1% (m/v) NaN$_3$ (streptavidin-alkaline phosphatase label) or 0.01% (w/v) Thimerosal (streptavidin-horseradish peroxidase label).

Next, this dilution series is tested in an immunohistochemical procedure to determine acceptable staining intensity. Briefly, paraffin-embedded tissue sections of 4-10 micron thickness are deparaffinated, hydrated and treated 5-10 minutes with 3% $H_2O_2$ (for horseradish peroxidase conjugate), washed and incubated with an appropriate primary antibody (i.e., murine monoclonal anti-insulin antibody was used with human pancreas sections at titer of 1:1000). After two 5 minute washes in PBS, the tissue is incubated with one of the dilutions of link complex for 20 minutes at room temperature. After two more 5 minute washes in PBS, the sections are further incubated for 20 minutes at room temperature in a solution of streptavidin-conjugated enzyme diluted in 0.01 M potassium phosphate buffer, (pH 7.6) containing 0.132 M NaCl, 1.0% bovine serum albumin, 0.008% Gentamycin, Neomycin, sulfate (Sigma; cat #N1876) and 0.01% (w/v) Thimerosal (Sigma; cat #T5125), (streptavidin-peroxidase conjugate) or 0.1% (w/v) sodium azide (streptavidin-alkaline phosphatase conjugate).

After two more 5 minute washes in PBS, the sections are stained with an appropriate substrate solution. For horseradish peroxidase, the solution contained 2.55 ml 0.1M sodium acetate/NaOH, pH 5.2, 0.096% (v/v) $H_2O_2$, to which 50 $\mu$l of 1.0M 3-amino-9-ethyl-carbazole in N.N demthyl formamide was added.

After counterstaining with hematoxylin, the intensity of staining is graded on a scale of 0 to 4.5 as described previously.

The most dilute concentration of link complex which still gave maximum staining (i.e., no decrease in staining compared to the most concentrated link complex was determined. Typically, the concentration was approximately 1 $\mu$g/ml for goat anti-mouse IgG and 3 $\mu$g/ml for rabbit anti-goat IgG Fc specific.

EXAMPLE III

Sensitivity of the Link Complex in Detecting Insulin in Normal Pancreas

The link reagent complex may be used to stain a wide variety of cells and tissue antigens in immunohistochemical procedures. The following example illustrates the use of the reagent complex for staining insulin in normal pancreas. In brief, paraffin-embedded sections of 5 micron thickness were deparaffinated and hydrated to water. Slides were incubated for 30 minutes with varying concentrations of monoclonal antibody to insulin. Following a quick rinse with PBS, the reagent complex or standard biotinylated link antibody (biotinylated goat anti-mouse IgG) for the ABC and B-SA method was applied to the tissue; incubation was for 20 minutes. The sections were then washed for 5 minutes in a bath of PBS. Next, a solution containing 1 ug/ml of streptavidin-alkaline phosphatase in PBS with 1% bovine serum albumin was added to each tissue section and incubated for 20 minutes. After a brief wash in PBS, color was developed for alkaline phosphatase as previously described. After counterstaining and coverslipping, the slides were graded as before on a scale of 0 to 4.5.

TABLE 2

| Staining Intensity of Reagent Complex Compared to B-SA and ABC Methods | | | |
|---|---|---|---|
| Dilution of Anti-Insulin Antibody | B-SA Method | ABC Method | Reagent Complex Method |
| 1:1000 | 4.5 | 4.5 | 4.5 |
| 1:2000 | 4.5 | 4.5 | 4.5 |
| 1:5000 | 3.5 | 3.5 | 4.5 |
| 1:10,000 | 3.0 | 3.0 | 4.5 |
| 1:20,000 | 3.0 | 2.0 | 4.5 |
| 1:40,000 | 2.0 | 2.0 | 4.5 |
| 1:80,000 | 1.0 | 1.0 | 4.5 |
| 1:125,000 | 0 | 0 | 4.0 |
| 1:250,000 | 0 | 0 | 3.5 |
| 1:500,000 | 0 | 0 | 3.0 |
| 1:1,000,000 | 0 | 0 | 3.0 |

For a general comparison, the reagent complex at a dilution of 1:1,000,000 of the primary antibody was about as sensitive as the standard link (B-SA and ABC methods) at a dilution of the primary antibody of 1:20,000. Therefore, the method employing the reagent complex was approximately 50 times more sensitive than either the B-SA or ABC methods.

EXAMPLE IV

Stability of the Link Complex

Product stability over a period of time is defined as equivalency of performance of the product after aging for that period of time to performance of the product at zero time; i.e., immediately after manufacture. Stability of a product stored at 4°C for a long period of time (e.g., one year) may be simulated by storing the product for a shorter time period at elevated temperatures (e.g., at 37°C for one week). (Kirkwood, TBL, J. Biol. Stand. (1984) 12:195-206). Stability of the reagent complex may be tested, for example, by comparing staining behavior of complex stored at 4°C for a length of time with that of complex stored at 37°C for the same length of time. One manner in which this is done is to manufacture a batch of reagent complex, aliquot into 1.0 ml portions and incubate either at 4°C or 37°C for 2, 3, and 4 weeks. The various aliquots are withdrawn after the designated period of time and used to stain one or more tissues. For example, slides of normal pancreas were stained with monoclonal antibody to insulin. The insulin antibody was applied to the tissue sections, and after 15 minutes of incubation, the slides were washed and an aliquot of the link complex was applied to each slide. Slides were incubated for 15 minutes and washed in a PBS bath for 5 minutes. A 1 $\mu$g/ml solution of streptavidin-alkaline phosphatase was then applied to each slide for 20 minutes. After a 5 minute wash in PBS, slides were then stained for 30 minutes with the substrate composed of naphthol AS-MX phosphate and Fast Red TR salt as previously described. Slides were counterstained, coverslipped and graded on a scale of 0 to 4.5 as defined previously. The results from that stability test are summarized below.

TABLE 3

| Staining Intensity of Insulin With Link Complex | | | | | | | |
|---|---|---|---|---|---|---|---|
| | | 2 weeks | | 3 weeks | | 4 weeks | |
| Link Complex Batch | Zero-time | 4° | 37° | 4° | 37° | 4° | 37° |
| A | 4.5 | 4.5 | 4.5 | 4.5 | 4.5 | 4.5 | 4.5 |
| B | 4.5 | 4.5 | 4.5 | 4.5 | 4.5 | 4.5 | 4.5 |

These results indicate that 1) there was no significant difference in the staining behavior between the two batches of reagent complexes; 2) the reagent complex tested as described above was stable at 4°C and 37°C for up to 4 weeks.

EXAMPLE V

Immunohistochemical Staining of Tissues With Link Reagent Complex

The link reagent complex was used with various monoclonal antibodies and compared to a standard B-SA method which utilized a conventional monomeric biotinylated link antibody. In the data below, the enzyme used was alkaline phosphatase; hence the method of staining involves the use of Fast Red TR salt and naphthol AS-MX phosphate as previously described.

TABLE 4

| Use of Link Complex In Immunohistochemical Staining | | | |
|---|---|---|---|
| | | Intensity Score | |
| Antibody | Tissue | Link Complex | B-SA |
| LCA | Tonsil | 4.5 | 3.0 |
| B-Cell | Tonsil | 4.5 | 1.0 |
| T-Cell | Tonsil | 4.5 | 3.0 |
| EMA | Breast Tumor | 4.5 | 3.0 |
| Progesterone-receptor | Breast Tumor | 4.0 | 0.0 |
| Estrogen-receptor | Breast Tumor | 4.5 | 1.0 |

These results indicate that in every case tested, the link complex method was superior to the standard B-SA method in the intensity of staining observed.

EXAMPLE VI

Use of the Link Complex In ELISA Procedures

A polyclonal antibody to luteinizing hormone (LH) was prepared in 0.1 M sodium carbonate, pH 9.5, at a concentration of 3 $\mu$g/ml. 50 $\mu$l of this solution was then added to each well of a 96-well polystyrene plate. Each plate was then incubated overnight at 4° C to allow the antibody to bind to the wells. Unbound sites in each well were then blocked by the addition of 50 $\mu$l of 1% bovine serum albumin in PBS containing 0.1% sodium azide. The plates were then incubated for 2 hours. Subsequent to this the plates were washed three times with wash buffer containing PBS and 0.1% Tween 20 (Sigma Chemical Co.). Plates were then stored at 4° C until needed.

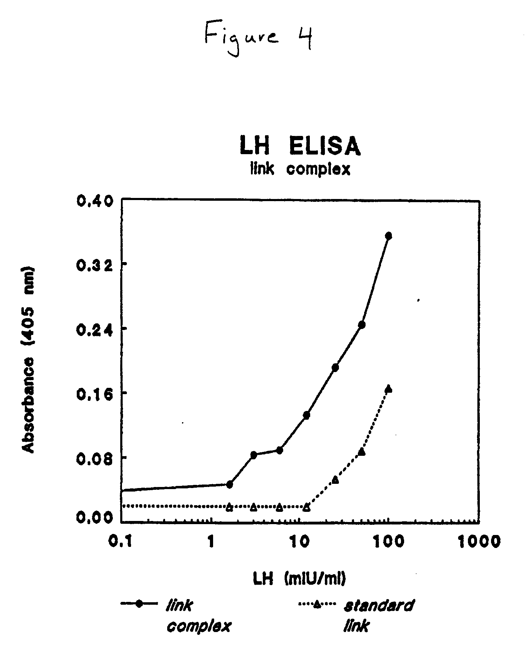

To perform the assay for quantitative measurement of LH, known concentrations of LH ranging from 100 mIU/ml to 0.1 mIU.ml were applied to the wells in a 50 $\mu$l volume. Any LH present in the sample would be captured by the anti-LH antibody attached to the well and would become immobilized within the well. After a 15 minute incubation, wells were rinsed twice in wash buffer. A monoclonal antibody to LH in a 50 $\mu$l volume was applied to each well for 15 minutes. If LH had become immobilized to the well, then the monoclonal antibody to LH would also become bound to the well. To detect bound anti-LH antibody, wells were rinsed twice with wash buffer, and link complex or standard biotinylated link antibody (biotinylated goat anti-mouse IgG) was applied in a 50 $\mu$l volume to each well. After 15 minutes wells were washed 5 times in wash buffer, and the substrate solution was applied. Substrate was 2 mg/ml of p-nitrophenylphosphate in 0.1 M diethanolamine buffer. 100 $\mu$l of substrate was added, and color was allowed to develop for 30 minutes followed by addition of 100 $\mu$l of stop solution composed of 5% ethylinediamine tetraacetic acid (EDTA). The color intensity of each well was measured spectrophotomethrically at 405 nm, and results were graphed as shown in Figure 4.

The results shown in Figure 4 indicate that the link complex was approximately 10 times more sensitive in the detection of LH than was the standard link antibody.

EXAMPLE VII

Reagent Complex Formed With Primary Antibody

Although all of the preceding examples have described a link complex which specifically binds to a primary antibody which had first been reacted with a specific analyte, it can be seen that the reagent complex could be formulated with the first antibody being a primary antibody specific for the analyte of interest, and the second antibody being essentially as previously described, that is an antibody which specifically binds an epitope on the Fc region of the first antibody. According to this embodiment, the primary complex would be reacted directly with the analyte of interest, thereby binding a detectable label, such as biotin, to the analyte without requiring an additional link step. The following example will serve to illustrate the use of the primary complex composed of a primary antibody.

A primary complex was prepared where the first binding pair was a monoclonal antibody to LH and the second binding pair was biotinylated goat anti-mouse IgG Fc specific. The concentration and ratio of pair 1 to pair 2 was .11 ug to .33 ug respectively in a 1.0 ml volume.

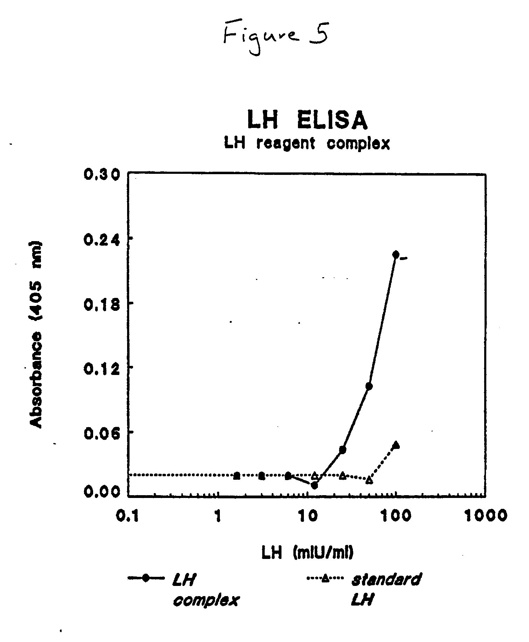

An ELISA assay was performed essentially as described in Example VI except that the primary complex was used in place of the primary antibody to LH. For comparison, a biotinylated primary antibody to LH was also tested. After incubation with the primary complex or primary antibody, the link step was omitted; all other steps remained the same. The results of this experiment are shown graphically in Figure 5.

These results indicate that the reagent complex can be substituted for the primary antibody with approximately a 10 fold enhancement in sensitivity for detection of LH antigen.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto.

**Claims**

1. A reagent complex suitable for use in analytical reactions using a detectable signal attached to a specific-binding reagent, comprising:
a first unlabelled antibody, wherein said first antibody has free specific-binding affinity for an analyte or an analyte-specific binding reagent; and
a second antibody having specific-binding affinity for an Fc region of said first antibody, said second antibody being bound to said Fc region to form said complex and being labelled with a detectable signal.

2. The reagent complex of claim 1, wherein said first antibody has specific-binding affinity for a non-antibody analyte.

3. The reagent complex of claim 1 or claim 2, wherein said second antibody and said first antibody are present in said complex in a ratio greater than 1:1.

4. The reagent complex of any one of claims 1 to 3, wherein more than 1 detectable signal is present on said second antibody.

5. In an assay that utilizes a specific-binding reaction between a detectably labelled analyte-specific binding reagent and its binding partner analyte to detect the presence of said analyte, an improvement which comprises:
utilizing a reagent complex to link said analyte to said label, wherein said complex comprises a first unlabelled antibody, wherein said first antibody has free specific-binding affinity for an analyte or an analyte-specific binding reagent, and a second antibody having specific-binding affinity for an Fc region of said first antibody, said second antibody being bound to said Fc region to form said complex and being labelled with a detectable signal.

6. The assay of claim 5, wherein more than 1 detectable signal is present on said second antibody.

7. The assay of claim 5 or claim 6, wherein said detectable signal is an enzyme, fluorophore, spin label, chemiluminescent or bioluminescent molecule, or radioisotope.

8. The assay of any one of claims 5 to 7, wherein said detectable signal is a biotin molecule or a third antibody.

9. The assay of claim 8, wherein said biotin molecule is further complexed with a multivalent avidin molecule that is covalently bound to at least one enzyme label.

10. The assay of claim 9, wherein said enzyme in alkaline phosphatase.

## FIGURE 1

**A. DIRECT COUPLING**

**B. INDIRECT COUPLING**

**C. PAP METHOD**

**D. ABC METHOD**

**E. B-SA METHOD**

= antibody

-E = enzyme label (covalently attached)

= covalently attached biotin

= avidin

FIGURE 2

A. LINK REAGENT COMPLEX CONTAINING ENZYME LABEL

$\lambda$ = goat anti-mouse IgG

$\lambda^E$ = rabbit anti-goat IgG Fc specific

-E = enzyme label

B. LINK REAGENT COMPLEX CONTAINING BIOTIN LABEL

$\lambda$ = goat anti-mouse IgG

$\lambda$ = rabbit anti-goat IgG Fc specific

-● = biotin label

C. PRIMARY ANTIBODY REAGENT COMPLEX

$\lambda$ = mouse anti-LH

$\lambda$ = goat anti mouse IgG Fc specific

-● = biotin label

D. BI-SPECIFIC REAGENT COMPLEX

$\lambda$ = mouse antibody to antigen 1

$\lambda$ = mouse antibody to antigen 2

$\lambda$ = goat anti-mouse IgG Fc specific

E. BI-FUNCTIONAL REAGENT COMPLEX

$\lambda$ = mouse antibody to antigen

$\lambda$ = mouse antibody to enzyme

$\lambda$ = goat anti-mouse IgG Fc specific

F. AMPLIFIED REAGENT COMPLEX

λ = goat anti-mouse IgG

λ = rabbit anti-goat IgG Fc specific

ϒ = dog anti-rabbit IgG Fc specific

● = biotin label

Figure 3. Use of reagent complexes in ELISAs

Figure 4

LH ELISA
link complex

Figure 5

LH ELISA

LH reagent complex